# EUROPEAN PATENT APPLICATION

(11) **EP 2 394 997 A1**
(43) Date of publication of application: **14.12.2011**
(21) Application number: 10738529.6
(22) Date of filing: 03.02.2010
(51) Int. Cl.: C07D 401/04, C07D 211/58

(54) **PROCESS FOR PREPARING 1-(4-PIPERIDINYL)BENZIMIDAZOLONE DERIVATIVES**

(30) Priority: 03.02.2009 JP 2009022834
(71) Applicant: Mitsubishi Tanabe Pharma Corporation, Osaka 541-8505 (JP)
(72) Inventor: IWAMURA, Hiroshi, Osaka-shi, Osaka 541-8505 (JP); TARAO, Yoshihiro, Osaka-shi, Osaka 541-8505 (JP)
(74) Representative: von Kreisler Selting Werner
(86) International application number: PCT/JP2010/051465
(87) International publication number: WO 2010/090198

(57) **Abstract**

Provision of an industrial method of producing a 1-(4-piperidinyl)benzimidazolone derivative in a high yield at a low cost.

A production method of 1-(4-piperidinyl)benzimidazolone derivative (1) or a salt thereof according to the following steps.
[step 1] a step of subjecting a piperidone compound (2) and an aniline compound (3) to a reductive amination reaction to give a compound (4)
[step 2] a step of reacting the compound (4) with di-t-alkyl dicarbonate or N,N'-disuccinimidyl carbonate to give the 1-(4-piperidinyl)benzimidazolone derivative (1) 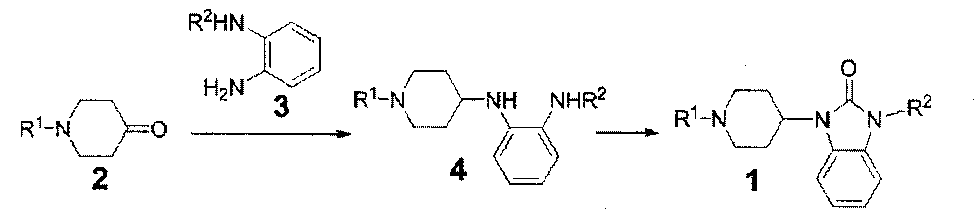 wherein R¹ is optionally substituted alkyl or an optionally substituted cyclic group, and
R² is optionally substituted alkyl, optionally substituted alkenyl or optionally substituted aryl.

## Description

### Technical Field

The present invention relates to a production method of a 1-(4-piperidinyl)benzimidazolone derivative useful as an intermediate for the synthesis of medicaments, and the like.

### Background Art

The 1-(4-piperidinyl)benzimidazolone derivative shows, for example, an ORL-1 (opioid receptor-like 1) receptor agonist activity, and is useful for the treatment of mental disorder, neuropathy and physiological disorder, particularly, amelioration of symptoms of anxiety and stress disorder, dysphoria, traumatic disorder, amnesia due to Alzheimer's disease or other dementia, epilepsy and convulsion, and acute and/or chronic pain symptom, symptoms of withdrawal from drug including drug withdrawal symptoms that occur during non-use of abused pharmaceutical products, or improvement of alcohol abuse, control of water balance, Na⁺ excretion, arterial blood pressure disorder and eating disorder such as obesity and anorexia, and circadian rhythm sleep disorder (patent documents 1, 2).
Patent documents 1 and 2 describe various production methods of 1-(4-piperidinyl)benzimidazolone derivatives. For example, for the production of a 1-(4-piperidinyl)benzimidazolone derivative having a substituted alkyl group at the 3-position, the method described includes subjecting a piperidone compound to a reductive amination reaction with phenylenediamine, followed by reaction with carbodiimidazole, alkylation, and further conversion of a substituent on alkyl. However, the methods are not industrially advantageous since they require many steps. To reduce the number of steps of these production methods, a production method of a convergence type is considered, wherein phenylenediamine is previously N-alkylated and then subjected to the reaction. However, it was not clear which of the two nitrogen atoms is involved in the reductive amination of N-alkylphenylenediamine, and whether the reaction proceeds selectively. In addition, only a few reports are present on a ureation reaction to follow. The reaction was indeed tried using diethyl carbonate, diphenyl carbonate, diethyl dicarbonate, carbodiimidazole and phenyl chlorocarbonate; however, the reaction did not proceed or side reaction occurred.

As a production method of the optically active amine shown below: 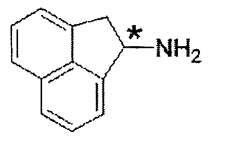 wherein * shows an asymmetric carbon, some methods have been reported.
In Example 11 of patent document 1, optically active amine is produced by subjecting ketone to borane-reduction in the presence of an asymmetric catalyst to lead to an optically active alcohol, which is then subjected to azidation and reduction. In non-patent document 1, optically active amine is produced by converting acenaphthene into dialkylboron by asymmetric hydroboration, and then converting the dialkylboron into amino. However, these production methods require expensive asymmetric catalysts, dangerous reagents and the like in large amounts, and are difficult for industrial use.
In addition, Scheme 3 of non-patent document 2 describes that high asymmetric induction occurred when an optically active 1-arylethylamine derivative was condensed with ketone and the obtained imine was reduced with sodium borohydride, and that when optically active phenylglycinol was used, a treatment with sodium periodate caused dissociation of 2-hydroxy-1-phenylethyl to give optically active amine. When this method was applied to acenaphthenone, however, attempts to isolate an intermediate imine caused by-production of a large amount of self-condensed dimmer of ketone, making it difficult to obtain the imine. Thus, the method could not be applied directly.

### [Document List]

### [patent documents]

patent document 1: W02003/082333
patent document 2: W02008/105497

### [non-patent documents]

non-patent document 1: Chem. Eur. J. 2006, 10, 1840
non-patent document 2: Org. Process. Res. Dev. 2007, 11, 539

### [Summary of Invention]

### Problems to be Solved by the Invention

The present invention aims to provide an industrial method of producing a 1-(4-piperidinyl)benzimidazolone derivative in a high yield at a low cost with a small number of steps.

### Means of Solving the Problems

The present inventors have conducted intensive studies in an attempt to solve the above-mentioned problems and found that, in a reductive amination of compound (3) obtained by introducing a substituent, R², of 1-(4-piperidinyl)benzimidazolone derivative (1) into phenylenediamine in advance with compound (2), the reaction surprisingly occurs only at a primary amino group with good selectivity to give compound (4), and furthermore, that 1-(4-piperidinyl)benzimidazolone derivative (1) or a salt thereof can be produced in an extremely high yield by using a particular carbonylation reagent, di-t-alkyl dicarbonate or N,N'-disuccinimidyl carbonate, which resulted in the completion of the present invention. 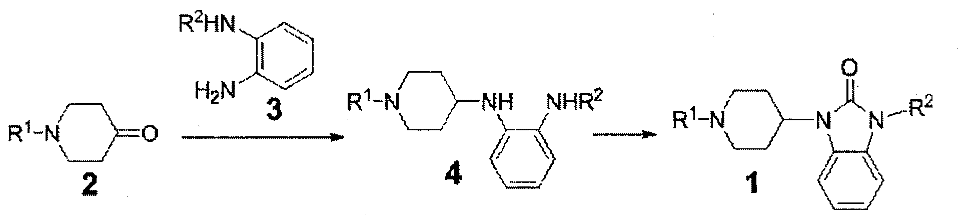
wherein R¹ is optionally substituted alkyl or an optionally substituted cyclic group, and
R² is optionally substituted alkyl, optionally substituted alkenyl or optionally substituted aryl.

Accordingly, the present invention is as described below.
[1] A method of producing a 1-(4-piperidinyl)benzimidazolone derivative of formula 1 or a salt thereof, comprising the following steps:
   [step 1] a step of subjecting a piperidone compound of formula 2 and an aniline compound of formula 3 to a reductive amination reaction to give a compound of formula 4 [step 2] a step of reacting the compound of formula 4 with dit-alkyl dicarbonate or N,N'-disuccinimidyl carbonate to give the 1-(4-piperidinyl)benzimidazolone derivative of formula 1 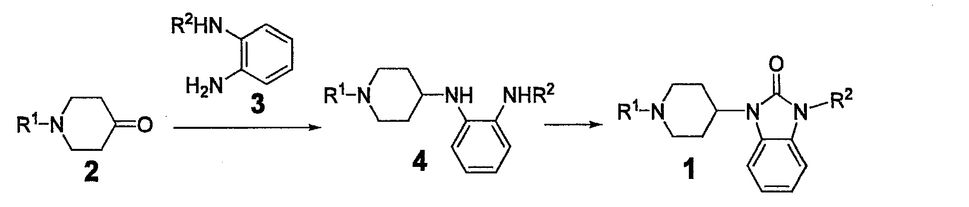
      wherein R¹ is optionally substituted alkyl or an optionally substituted cyclic group, and
      R² is optionally substituted alkyl, optionally substituted alkenyl or optionally substituted aryl.

[2] The production method of [1], wherein R¹ is any group of the following formulas (a) - (c): 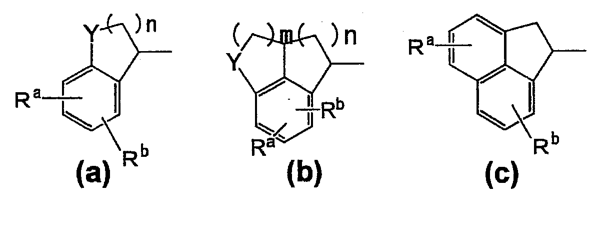
   wherein m and n are the same or different and each is an integer of 1 - 3,
   R^{a} and R^{b} are the same or different and each is hydrogen, alkyl, halogen, alkoxy, trifluoromethyl, trifluoromethoxy, hydroxyl, nitro, amino, alkanoylamino or cyano, and
   Y is CH₂, C(CH₃)₂, O, S, SO or SO₂, and
   R² is alkyl, alkenyl, alkyl-carboxyl, alkyl-C(O)O-alkyl, alkenyl-C(O)0-alkyl, alkyl-O-alkyl, alkyl-C(O)-NR³R⁴, alkyl-S-alkyl, alkyl-S(O)-alkyl, alkyl-S(O)₂-alkyl, alkyl-S(O)-NR³R⁴, alkyl-NR³R⁴, alkyl-NR⁵-C(O)-alkyl, phenyl (which is optionally substituted by alkyl, halogen, alkoxy, phenoxy or benzyloxy), or benzyl (benzene ring in benzyl is optionally substituted by alkyl, halogen, alkoxy, phenoxy or benzyloxy)
   wherein R³ and R⁴ are the same or different and each is hydrogen, alkyl (which is optionally substituted by C₃-C₇ cycloalkyl), cycloalkyl or alkenyl, or R³ and R⁴ may be bonded to form, together with the adjacent nitrogen atom, a saturated nitrogen-containing heterocycle (which is optionally substituted by alkyl, halogen, alkoxy, phenoxy or benzyloxy), and
   R⁵ is hydrogen, alkyl or alkenyl.
[3] The production method of [1], wherein R¹ is acenaphthen-1-yl and R² is N-methylcarbamoylmethyl.
[4] The production method of any of [1] to [3], wherein a reducing agent selected from alkali borohydride, alkali triacyloxyborohydride and borane-amine complex, and an acid selected from formic acid, alkanoic acid, halogenated alkanoic acid, arylalkanoic acid, optionally substituted arylcarboxylic acid, optionally substituted alkylsulfonic acid and optionally substituted arylsulfonic acid are used in the reductive amination reaction of step 1.
[5] The production method of any of [1] to [4], wherein, in step 2, the compound of formula 4 is reacted with di-t-butyl dicarbonate to give a 1-(4-piperidinyl)benzimidazolone derivative of formula 1.
[6] The production method of any of [1] to [5], wherein, in step 2, a base selected from pyridine, dimethylaminopyridine and N-methylimidazole is used.
[7] The production method of any of [1] to [6], wherein the reaction of step 2 is performed in a carbon dioxide atmosphere.
[8] A compound represented by the formula: 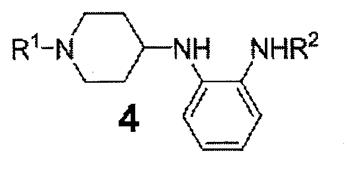 wherein R¹ and R² are as defined in [2],
   or a salt thereof.
[9] The compound of [8], wherein R¹ is acenaphthen-1-yl and R² is N-methylcarbamoylmethyl, or a salt thereof.
[10] A method of producing a 1-(4-piperidinyl)benzimidazolone derivative of formula 1a or a salt thereof, comprising the following steps:
   [step 1] a step of reacting a ketone of formula 6 with an optically active aminoalcohol compound of formula 7 to form imine in the reaction system, and reducing the imine to give a compound of formula 8
   [step 2] a step of reacting the compound of formula 8 with an oxidant to give an optically active amine of formula 5
   [step 3] a step of treating the optically active amine of formula 5 with N-ethyl-N-methyl-4-oxopiperidinium iodide to give a piperidone compound of formula 2a
   [step 4] a step of subjecting the piperidone compound of formula 2a and an aniline compound of formula 3 to a reductive amination reaction to give a compound of formula 4a [step 5] a step of reacting the compound of formula 4a with di-t-alkyl dicarbonate or N,N'-disuccinimidyl carbonate to give the 1-(4-piperidinyl)benzimidazolone derivative of formula 1a 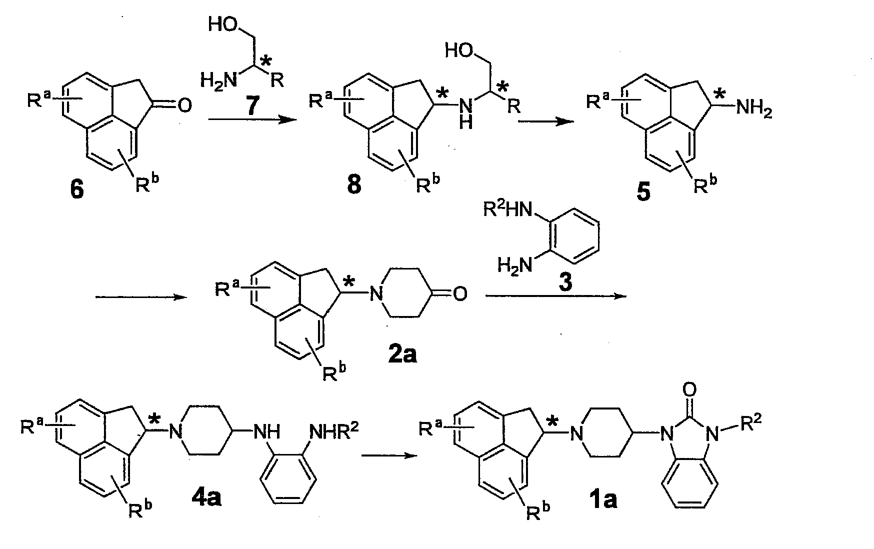
      wherein R is optionally substituted C₃-C₁₀ alkyl, optionally substituted phenyl or optionally substituted benzyl,
      * shows an asymmetric carbon, and
      R², R^{a} and R^{b} are as defined in [2].

### [Description of Embodiments]

Examples of alkyl include straight chain or branched chain C₁-C₆ alkyl, such as methyl, ethyl, propyl, isopropyl, butyl, isobutyl, secondary butyl, tertiary butyl, pentyl, hexyl and the like. Preferred is straight chain or branched chain C₁-C₄ alkyl, and more preferred are methyl, ethyl, propyl and isopropyl.
Examples of alkenyl include straight chain or branched chain C₂-C₆ alkenyl, such as vinyl, 1-propenyl, 2-propenyl, isopropenyl, 1-butenyl, 2-butenyl, 3-butenyl and the like. Preferred is straight chain or branched chain C₂-C₄ alkenyl.
Examples of halogen include chlorine, iodine, fluorine and bromine. Preferred are chlorine, fluorine and bromine, and particularly preferred is fluorine.
Examples of alkoxy include straight chain or branched chain C₁-C₆ alkoxy such as methoxy, ethoxy, propoxy, isopropoxy, butoxy, isobutoxy, secondary butoxy, tertiary butoxy, pentyloxy, hexyloxy and the like. Preferred is straight chain or branched chain C₁-C₄ alkoxy, and particularly preferred is methoxy.
Examples of alkanoyl include C₂-C₆ alkanoyl such as acetyl, propionyl, butyryl, pentanoyl and the like. Preferred is acetyl.
Examples of the cycloalkyl include C₃-C₇ cycloalkyl such as cyclopropyl, cyclobutyl, cyclopentyl, cyclohexyl, cycloheptyl and the like. Preferred is cyclopropyl.
Examples of aryl include C₆-C₁₀ aryl such as phenyl, 1-naphthyl, 2-naphthyl and the like. Preferred is phenyl.

Examples of the cyclic group include a saturated or unsaturated, monocyclic or fused cyclic hydrocarbon ring group or heterocyclic group having 3 to 17 ring constituting atoms.
Here, examples of the monocyclic hydrocarbon ring group include C₃-C₇ cycloalkyl, C₆-C₁₀ aryl and the like.
Here, examples of the monocyclic heterocyclic group include a 4- to 7-membered monocyclic saturated heterocyclic group containing, as ring-constituting atom besides carbon atom, 1 to 4 hetero atoms selected from a nitrogen atom, an oxygen atom and a sulfur atom (the carbon atom and sulfur atom constituting the ring may be oxidized) (e.g., a pyrrolidinyl group, a tetrahydrofuryl group, a tetrahydropyranyl group, an oxepanyl group, a tetrahydrothienyl group, a tetrahydrothiopyranyl group, a thiepanyl group, an oxazolidinyl group, an isoxazolidinyl group, a thiazolidinyl group, an isothiazolidinyl group, an imidazolidinyl group, a pyrazolidinyl group, a piperidyl group, a morpholinyl group, a thiomorpholinyl group, a piperazinyl group, an azepanyl group, an azocanyl group etc.); an unsaturated or partially unsaturated heterocyclic group (e.g., a furyl group, a thienyl group, a pyrrolyl group, an oxazolyl group, an oxazolinyl group, an isoxazolyl group, an isoxazolinyl group, a thiazolyl group, a thiazolinyl group, an isothiazolyl group, an isothiazolinyl group, an imidazolyl group, an imidazolinyl group, a pyrazolyl group, a pyrazolinyl group, an oxadiazolyl group, a thiadiazolyl group, a triazolyl group, a tetrazolyl group, a pyridyl group, a pyrimidinyl group, a pyridazinyl group, a pyrazinyl group, a triazinyl group, a dihydropyridyl group etc.) and the like.
Here, examples of the fused cyclic hydrocarbon ring group or heterocyclic group include a fused cyclic group formed by a two or three rings selected from the above-mentioned "monocyclic hydrocarbon ring group" and "monocyclic heterocyclic group" and the like.
In the present specification, the number of the substituents that the group described as being "optionally substituted" may have is not limited as long as they are substitutable, and preferred is 1 to 3. When two or more substituent groups are present, they may be the same or different.

Examples of the substituent of the "alkyl" of the "optionally substituted alkyl" for R¹ include hydroxyl, alkoxy, halogen, alkanoyl, optionally substituted amino, nitro, cyano, aryl, cyclic group and the like, and one or more substituents can be present.
Examples of the substituent of the "cyclic group" of the "optionally substituted cyclic group" for R¹ include hydroxyl, alkoxy optionally substituted by 1 to 3 halogen, halogen, alkanoyl, amino optionally mono- or di-substituted by alkanoyl, nitro, cyano, aryl, alkyl optionally substituted by 1 to 3 halogen, a cyclic group and the like, and one or more substituents can be present.
Preferable examples of R¹ include a group of the formula (a) wherein Y is CH₂ and n is 2 and the group of formula (c), both mentioned above.

Examples of the saturated nitrogen-containing heterocycle formed by R³ and R⁴ bonded to each other and together with the adjacent nitrogen atom include a 5- or 6-membered ring containing, at least one nitrogen atom, and further optionally containing 1 to 3 hetero atoms selected from a nitrogen atom, an oxygen atom and a sulfur atom (the carbon atom and sulfur atom constituting the ring may be oxidized). Examples thereof include piperidine, pyrrolidine, morpholine, thiomorpholine, piperazine, thiazolidine, 2,4-dioxothiazolidine and the like. Preferred are piperazine, morpholine, pyrrolidine and 2,4-dioxothiazolidine, and particularly preferred are pyrrolidine and 2,4-dioxothiazolidine.

The salt of 1-(4-piperidinyl)benzimidazolone derivative (1) is not particularly limited as long as it is a pharmaceutically acceptable salt, and examples thereof include salts with inorganic acid (hydrochloric acid, hydrobromic acid, sulfuric acid, phosphoric acid, nitric acid etc.) or organic acid (acetic acid, propionic acid, succinic acid, glycolic acid, lactic acid, malic acid, tartaric acid, citric acid, maleic acid, fumaric acid, methanesulfonic acid, benzenesulfonic acid, p-toluenesulfonic acid, camphorsulfonic acid, ascorbic acid etc.). In addition, 1-(4-piperidinyl)benzimidazolone derivative (1) and these salts may be a solvate with water or an organic solvent.

The 1-(4-piperidinyl)benzimidazolone derivative (1) or a salt thereof can be produced by the following steps:
[step 1] a step of subjecting piperidone compound (2) and aniline compound (3) to a reductive amination reaction to give compound (4)
[step 2] a step of reacting compound (4) with di-t-alkyl dicarbonate or N,N'-disuccinimidyl carbonate to give 1-(4-piperidinyl)benzimidazolone derivative (1) 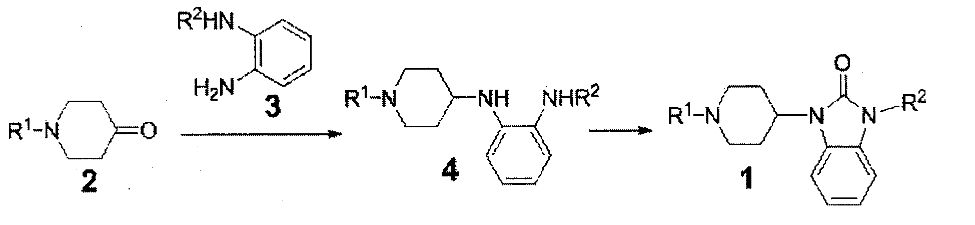 wherein R¹ and R² are as defined above.

In the production method of the present invention, an efficient production may be performed by, for example, introducing a protecting group into a functional group as necessary and removing the protecting group in a later step, and the like.
In addition, in each step, a work-up treatment after reaction may be performed by a conventional method, and isolation and purification can be performed by appropriately selecting or combining, where necessary, a conventional method such as crystallization, recrystallization, distillation, partitioning, silica gel chromatography, preparative HPLC and the like.

In step 1, compound (4) can be produced by subjecting piperidone compound (2) and aniline compound (3) to a reductive amination reaction. While piperidone compound (2) sometimes has, depending on the compound, a structure wherein the ketone moiety is hydrated, the compound can be used for the reaction in the same manner.
The reductive amination reaction can be performed by any generally known method. For example, the reaction can be carried out by using a particular reducing agent in the presence of an acid.
Examples of the acid include organic acids such as formic acid, alkanoic acid (e.g., acetic acid, propionic acid, butanoic acid, pentanoic acid, heptanoic acid etc.), halogenated alkanoic acid (e.g., chloroacetic acid, dichloroacetic acid, trichloroacetic acid, trifluoroacetic acid, fluoroacetic acid, difluoroacetic acid etc.), arylalkanoic acid, optionally substituted arylcarboxylic acid (e.g., benzoic acid), optionally substituted alkylsulfonic acid (e.g., methanesulfonic acid etc.), optionally substituted arylsulfonic acid (e.g., p-toluenesulfonic acid, benzenesulfonic acid etc.) and the like, inorganic acids such as titanium tetraisopropoxide and the like. The acid can be used in, for example, 0.1 - 100 mol, preferably 0.8 - 20 mol, per 1 mol of piperidone compound (2).
Examples of the particular reducing agent include alkali borohydride (e.g., sodium borohydride etc.), alkali triacyloxyborohydride (e.g., sodium triacetoxyborohydride etc.), borane-amine complex (e.g., dimethylamine borane, triethylamine borane, trimethylamine borane, t-butylamine borane, N,N-diethylaniline borane, 2-picoline borane etc.) and the like. The reducing agent can be generally used in 0.8 - 100 mol, preferably 0.8 - 2 mol, per 1 mol of piperidone compound (2).
The amount of aniline compound (3) to be used is appropriately selected and, for example, 0.8 - 2 mol can be used per 1 mol of piperidone compound (2). In addition, as the reaction solvent, dichloromethane, 1,2-dichloroethane, methanol, ethanol, tetrahydrofuran (THF), acetonitrile, 2-propanol, toluene, dimethylformamide (DMF), 1,3-dimethylimidazolidinone and the like can be used.
The reaction temperature can be generally optionally selected from -20 to 100°C, and the reaction time is generally about 10 min - 1 day.
Aniline compound (3) can be produced in the same manner as in the method described in J. Med. Chem. 1994, 37, 758, for example, by reducing a 1-substituted amino-2-nitrobenzene derivative.

In step 2, 1-(4-piperidinyl)benzimidazolone derivative (1) can be produced by reacting compound (4) with di-t-alkyl dicarbonate or N,N'-disuccinimidyl carbonate, and di-t-alkyl dicarbonate is preferably used.
Preferable examples of di-t-alkyl dicarbonate include dit-butyl dicarbonate. The di-t-alkyl dicarbonate or N,N'-disuccinimidyl carbonate can be used in, for example, 0.8 - 100 mol, preferably 0.8 - 5 mol, per 1 mol of compound (4).
In this reaction, a base is preferably added. In the case of, for example, di-t-alkyl dicarbonate, addition of a base can promote progress of the reaction. Examples of the base include pyridine, N,N-dimethyl-4-aminopyridine, N-methylimidazole, imidazole and the like. Preferred are pyridine, N,N-dimethyl-4-aminopyridine and N-methylimidazole, more preferred is 1-methylimidazole. The amount of the base to be used is, for example, 0.05 - 10 mol, preferably 0.2 - 2 mol, per 1 mol of compound (4).
Examples of the reaction solvent include dichloromethane, 1,2-dichloroethane, methanol, ethanol, THF, acetonitrile, isopropanol, toluene, DMF, dimethylimidazolidinone and the like.
The reaction temperature can be generally selected from - 20 to 100°C, and the reaction time is generally about 10 min - 1 day.
In addition, this step is preferably performed in a carbon dioxide atmosphere since the yield is stable.

Piperidone compound (2), the starting material, is a known compound or can be easily produced according to a generally known method. In addition, piperidone compound (2a), which is piperidone compound (2) wherein R¹ is an optically active group of formula (c), can be derived from, for example, optically active amine (5): 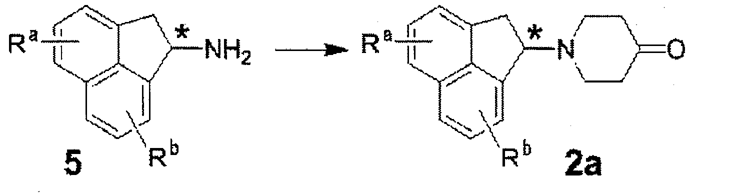 wherein R^{a} and R^{b} are as defined above, and * shows an asymmetric carbon.
Piperidone compound (2a) can be produced by treating optically active amine (5) with N-ethyl-N-methyl-4-oxopiperidinium iodide. The amount of N-ethyl-N-methyl-4-oxopiperidinium iodide to be used is, for example, 0.9 - 1.1 mol, preferably about equimol, per 1 mol of piperidone compound (2a).
As the reaction solvent, water, alcohol (ethanol etc.), acetonitrile and the like can be used alone or in a mixture. It is also preferable to use a base such as potassium carbonate, sodium hydroxide and the like in 0.5 - 2 molar equivalents. The reaction temperature is 0°C to 100°C, preferably 40°C to 90°C, and the reaction time is generally about 10 min - 1 day.

Optically active amine (5) can be produced from, for example, ketone (6) via the following production method or intermediate in a high yield at a high optical purity.
[Production method A-1] Production method of optically active amine (5) containing the following steps:
[step 1] a step of reacting ketone (6) with optically active aminoalcohol compound (7) to form imine in the reaction system, and reducing the imine to give compound (8)
[step 2] a step of reacting compound (8) with an oxidant to give optically active amine (5) 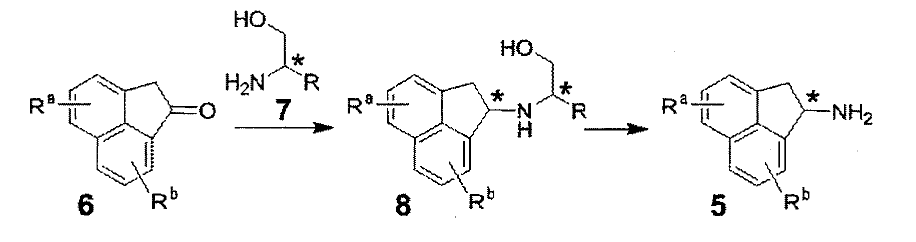
wherein R, R^{a}, R^{b} and * are as defined above.
[Production method A-2] Production method described in [Production method A-1] wherein R is isopropyl, isobutyl or tertiary butyl
[Production method A-3] Production method described in [Production method A-1] or [Production method A-2], wherein alkali triacetoxyborohydride or amine borane is used as an imine reducing agent in step 1
[Production method A-4] Production method described in any of [Production method A-1] to [Production method A-3], wherein orthoperiodic acid, metaperiodic acid or periodate is used as an oxidant in step 2

[Intermediate B-1] A compound represented by formula 8: 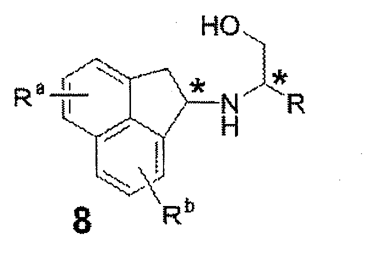 wherein R, R^{a}, R^{b} and * are as defined above, or a salt thereof.
[Intermediate B-2] Any of the following compounds or a salt thereof:
2-{(1R)-(acenaphthen-1-yl)amino}-(2S)-4-methylpentan-1-o1
2-{(1R)-(acenaphthen-1-yl)amino}-(2S)-3-methylbutan-1-ol
2-{(1R)-(acenaphthen-1-yl)amino}-(2S)-3,3-dimethylbutan-1-ol

In step 1 of production method A-1, ketone (6) is reacted with optically active aminoalcohol compound (7) to form imine in the reaction system, and reducing the imine, which induces high asymmetric induction to give compound (8) in a high yield. By performing reduction reation without isolation of imine, while producting the imine, by-production of a dimer of ketone (6) due to self-condensation is prevented, thus first enabling to achieve a high yield.
In the production of imine, the reaction proceeds even in the absence of an acid catalyst. However, addition of an acid catalyst is preferable. Examples of the acid catalyst include formic acid, acetic acid, propionic acid, isobutyric acid, hexanic acid, tosylic acid, benzoic acid and the like, and they can be used in 0 - 10 molar equivalents relative to ketone (6). For example, 0.5 - 1.5 molar equivalents are preferable for formic acid, and 2 - 8 molar equivalents are preferable for acetic acid.
In optically active aminoalcohol compound (7), examples of the substituent of the "C₃-C₁₀ alkyl" of the "optionally substituted C₃-C₁₀ alkyl" for R include hydroxyl and the like, examples of the substituent of the "phenyl" of the "optionally substituted phenyl" for R include hydroxyl, alkyl, halogen and the like, and examples of the substituent of the "benzyl" of the "optionally substituted benzyl" for R include hydroxyl, halogen and the like. The number of these substituents is not limited as long as they are substitutable, and preferred is 1 to 3. When two or more substituents are present, they may be the same or different. Preferable examples of optically active aminoalcohol compound (7) include optically active forms such as 2-phenylglycinol, valinol, leucinol, isoleucinol, t-leucinol, 2-amino-1-hexanol and the like, more preferred are optically active forms of valinol, leucinol and t-leucinol. They can be added in 1 - 4 molar equivalents relative to ketone (6). When, for example, (R)-(acenaphthen-1-yl)amine is produced as optically active amine (5), (S)-forms such as (S)-valinol, (S)-leucinol and the like are used.
As the reducing agent of imine, alkali triacetoxyborohydride, various amine boranes (2-picoline borane, 5-ethyl-2-methylpyridine borane, pyridine borane, t-butylamine borane, diethylisopropylamine borane etc.) can be used in 1 - 5 molar equivalents relative to ketone (6). Preferable examples of amine borane include 2-picoline borane, 5-ethyl-2-methylpyridine borane and pyridine borane.
As the reaction solvent, for example, THF, t-butyl methyl ether, heptane, toluene, methanol, ethanol, 2-propanol, N-methylpiperidone, 1,3-dimethyl-2-imidazolidinone and the like can be used. Preferably, they are used in about 2 - 10 mL/g relative to ketone (6). The reaction temperature is 20°C - 80°C, preferably 30°C - 70°C. The reaction time is generally about 10 min - 1 day. Molecular sieves, magnesium sulfate, sodium sulfate, sodium acetate and the like may be added as a dehydrating agent.

In step 2 of production method A-1, compound (8) is reacted with an oxidant to give optically active amine (5). As the oxidant, orthoperiodic acid, metaperiodic acid, periodate, lead tetraacetate and the like can be used, and preferable examples include orthoperiodic acid and periodate. The oxidant is preferably used in 1 - 3 molar equivalents relative to compound (8).
As the reaction solvent, water or a mixed solvent of water and an organic solvent (methanol, acetonitrile, toluene etc.) can be used, and it is preferably used in about 5 - 30 mL/g relative to compound (8). The reaction temperature is at -10°C to 30°C, preferably 0°C to 20°C. The reaction time is generally 10 min - 1 day. In the reaction, 2 - 15 molar equivalents of a primary amine such as methylamine and the like may be co-present relative to compound (8) .
When optically active amine (5) is derived from, for example, N-(acenaphthen-1-yl)-(1-phenylethyl)amine derivative and the like, undesirable elimination or saturation and the like proceed, which prevents production of optically active amine (5) in a good yield. In the production method of the present invention, an elimination reaction can be performed extremely mildly using an oxidant such as orthoperiodic acid and the like via compound (8) having a 2-hydroxylethylamine structure, whereby optically active amine (5) can be obtained in a high yield.

### Examples

The present invention is explained in more detail in the following by referring to Example and the like, which are not to be construed as limitative of the scope of the present invention.

### Example 1

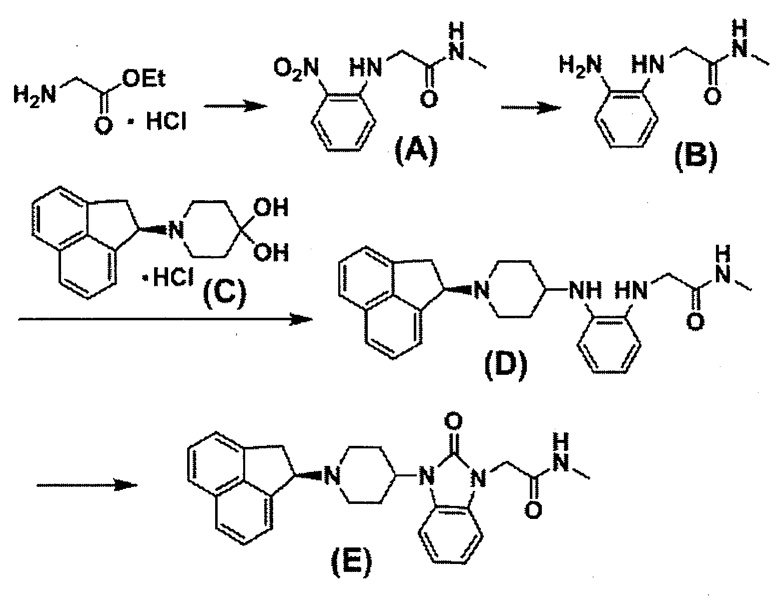

### Production of compound (A)

To a solution (125 mL) of glycine ethyl ester hydrochloride (140 g, 1.00 mol) in water was added 40% aqueous methylamine solution (206 g, 2.65 mol) over 10 min at room temperature, and the mixture was stirred at room temperature for 1 hr. 25% Aqueous sodium hydroxide solution (170 g) was added to the reaction mixture, and the solution was evaporated under reduced pressure. Ethanol (553 g) was added to the residue, and the mixture was filtered. The solution was evaporated under reduced pressure, and 1,3-dimethyl-2-imidazolidinone (795 g) and potassium carbonate (138 g, 0.999 mol) were added to the residue. To the solution was added dropwise a solution of 2-fluoronitrobenzene (114.1 g, 0.809 mol) in 1,3-dimethyl-2-imidazolidinone (371 g) over 10 min at room temperature, and the mixture was heated to 80°C and stirred for 5 hr. The reaction mixture was cooled, water (1100 mL) was added, and the mixture was stirred at 10 to 15°C for 1 hr. The obtained precipitate was filtered, washed with 1/1 (w/w) ethanol-water solution (900 g), and dried to give 2-(2-nitrophenylamino)-N-methylacetamide [compound (A)] (138.5 g, yield from 2-fluoronitrobenzene 82%).
¹H NMR (CD₃OD); 2.78 (3H, s), 4.03 (2H, s), 6.74 (2H, m), 7.50 (1H, m), 8.16 (1H, m)

### Production of compound (B)

To a suspension of compound (A) (12 g, 57.4 mmol) and ethanol (118 g) was added 5% Pd-C 50% water-containing product (2.4 g), and the reaction container was purged with nitrogen. After hydrogen purge, the mixture was heated to 50°C and stirred for 5 hr under a hydrogen atmosphere. The reaction mixture was cooled, filtered, and washed with ethanol (118 g). The filtrate was concentrated under reduced pressure, and dichloromethane (135 g) was added. The mixture was further concentrated under reduced pressure to give 2-(2-aminophenylamino)-N-methylacetamide [compound (B)] (10.8 g, quantitative).
¹H NMR (CDCl₃); 2.80 (3H, s), 3.81 (2H, s), 6.55 (1H, m), 6.70 (1H, m), 6.77 (2H, m), 6.85 (1H, m)

### Production of compound (D)

To a solution of compound (B) (33.5 g, 187 mmol) in dichloromethane (520 g) was added compound (C) produced in Example 3 (52 g, 170 mmol), and the mixture was stirred at 20 to 24°C. Acetic acid (107.22 g, 1785 mmol) was added dropwise, and the mixture was stirred at the same temperature for 1 hr. The liquid prepared here was added dropwise to a solution of t-butylamine borane (14.81 g, 170 mmol) in dichloromethane (500 g) at 10 to 20°C, and the mixture was stirred at the same temperature for 3 hr. The reaction mixture was added dropwise to a mixture of 20% sodium hydroxide (477 g) and water (142 g) at 16 to 19°C, and the mixture was stirred, left standing and partitioned. Activated carbon (41.94 g) was added to the organic layer, and the mixture was passed through a 1.0 µm membrane filter. Acetonitrile (330 g) was added, and the solvent was evaporated to about 370 g under reduced pressure (HPLC quantitative yield from compound (C) 85.9%). The reaction mixture was partially collected and purified by silica gel column chromatography to give 2-(2-(1-((R)-acenaphthen-1-yl)piperidin-4-ylamino)phenylamino)-N-methylacetamide [compound (D)] and the analysis data.
¹H NMR (CDCl₃); 1.55 (2H, m), 2.00 (2H, m), 2.35 (1H, m), 2.51 (1H, m), 2.70 (1H, m), 2.80 (3H, s), 2.90 (1H, m), 3.20 (1H, m), 3.45 (2H, m), 3.78 (2H, s), 4.20 (1H, m), 4.98 (1H, m), 6.60 (2H, m), 6.78 (3H, m), 7.30 (2H, m), 7.50 (3H, m), 7.60 (1H, d, J = 8.0 Hz), 7.70 (1H, d, J = 8.0 Hz)

### Production of compound (E)

To a solution of compound (D) (60.53 g, 146 mmol) in acetonitrile (about 300 mL) was added N-methylimidazole (3.60 g, 43.8 mmol). A solution of di-t-butyl dicarbonate (44.61 g, 204 mmol) in acetonitrile (121 g) was added at 29 to 31°C, and the mixture was stirred at the same temperature for 4 hr. The reaction mixture was cooled to 2 to 5°C, and the suspension was aged for 1 hr. The crystals were collected by filtration and washed with acetonitrile (400 g). The crystals were dried at 45 to 55°C to give (R)-2-{3-[1-(acenaphthen-1-yl)piperidin-4-yl]-2,3-dihydro-2-oxobenzimidazol-1-yl}-N-methylacetamide [compound (E)] (42.47 g, yield from compound (C) 56.7%).
¹H NMR (CDCl₃); 1.75 (2H, m), 2.50 (4H, m), 2.80 (4H, m), 3.05 (1H, m), 3.41 (2H, m), 4.40 (1H, m), 4.49 (2H, s), 4.99 (1H, m), 6.11 (1H, m), 7.11 (3H, m), 7.31 (2H, m), 7.52 (3H, m), 7.63 (1H, m), 7.71 (1H, m)

### Example 2

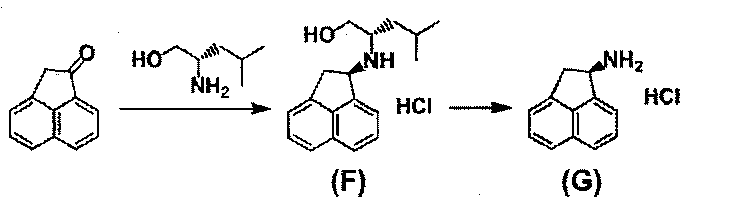

### Production of compound (F)

A mixture of acenaphthenone (2.06 g, 12.2 mmol), L-leucinol (1.97 g, 16.8 mmol), 2-picoline borane (2.18 g, 20.4 mmol), acetic acid (4.4 ml, 77.3 mmol) and THF (12 ml) was stirred at about 50°C for 10 hr. The reaction solution was cooled, and 6M hydrochloric acid (14 ml) was slowly added dropwise. After the completion of the dropwise addition and termination of gas generation, toluene (10 ml), THF (5 ml) and aqueous sodium hydroxide solution (prepared from NaOH (about 10 g) and water (about 45 ml)) were added, and the mixture was partitioned. The aqueous layer was extracted with toluene (15 ml), the organic layers were combined and washed with 25% brine, and concentrated hydrochloric acid (1.4 ml) was added. 2-Propanol (15 ml) was added to the obtained suspension. The solvent was evaporated at 60°C to about half amount. 2-Propanol (4 ml) was added, and the mixture was stirred for about 1 hr under ice-cooling. The mixture was filtered, washed with toluene, and vacuum dried to give the desired compound (2.37 g, yield 63%, diastereomer ratio 99.7:0.3). Furthermore, the filtrate was concentrated and crystallized to give 2-{(1R)-(acenaphthen-1-yl)amino}-(2S)-4-methylpentan-1-ol 1 hydrochloride [compound (F)] (0.61 g, yield 16%, diastereomer ratio 98.4:1.6).
¹H-NMR (DMSO-d6); 0.78(3H, d), 0.88(3H, d), 1.3-1.6(1H, m), 1.6-1.8(2H, d), 3.5-4.0(4H, m), 5.4-5.6(2H, m), 7.3-8.1(6H, m), 9.2-9.4(1H, brs), 9.4-9.7(1H, brs)
LC-MS: M+1 = 270

### Production of compound (G)

To a suspension of compound (F) (500 mg, 1.64 mmol) and methanol (1.5 ml) was added 40% methylamine-methanol solution (1.1 ml). Aqueous orthoperiodic acid solution (prepared by dissolving H₅IO₆ (957 mg, 4.20 mmol) in water (5 ml)) was added dropwise thereto over 15 min under ice-cooling, and the mixture was washed with water (1 ml). The mixture was allowed to warm gradually to room temperature, and left standing overnight. Water (2 ml) was added to the reaction mixture, and the mixture was extracted with toluene (8 ml). The aqueous layer was extracted twice with toluene (2 ml), and the organic layers were combined and concentrated to about 5 ml under reduced pressure. 6M Hydrochloric acid (4 ml) and methanol (about 8 ml) were added thereto, and the mixture was stirred at 30 to 40°C for about 10 min. The lower layer of the obtained two-layer solution was obtained by partitioning, and the upper layer was extracted with water/methanol=1/1 (4 ml) and 6M hydrochloric acid/methanol=1/1 (2 ml). The lower layers were combined, and methanol was mostly evaporated under reduced pressure. Water (10 ml), sodium hydroxide and toluene (7 ml) were added and the alkaline mixture was partitioned. The aqueous layer was extracted twice with toluene (about 2 ml). 2-Propanol (2 ml) was added to the toluene layer, and concentrated hydrochloric acid (0.2 ml) was added. The mixture was stirred to allow precipitation of a solid. 2-Propanol was mostly concentrated under reduced pressure, 2-propanol (about 0.2 ml) was added, and the mixture was stirred for about 40 min under ice-cooling. The solid was collected by filtration, and dried under reduced pressure at 80°C for about 1 hr to give the desied compound (253 mg, yield 75%, R/S = 98.8/1.2). 2-Propanol (7.5 ml) and water (0.75 ml) were added thereto, and the mixture was dissolved by heating under reflux, and about 5 ml was distilled away under reduced pressure at 50°C. Furthermore, 2-propanol (about 5 ml) was added, the operation of distillation of about 5 ml under reduced pressure at 50°C was repeated 3 times and the mixture was allowed to cool to room temperature. After stirring under ice-cooling for 30 min, the precipitated solid was collected by filtration, washed with 2-propanol, and dried under reduced pressure to give (R)-(acenaphthen-1-yl)amine 1 hydrochloride [compound (G)] (219 mg, recrystallization yield 87%, 99.88%ee) as a solid.
¹H-NMR (DMSO-d₆); 3.3-3.5 (1H, m), 3.8-4.0(1H, m), 5.1-5.3(1H, m), 7.3-7.5 (1H, m), 7.5-7.7 (2H, m), 7.7-7.8 (1H, m), 7.8-8.0 (2H, m), 8.7-9.2(3H, brs)

### Example 3

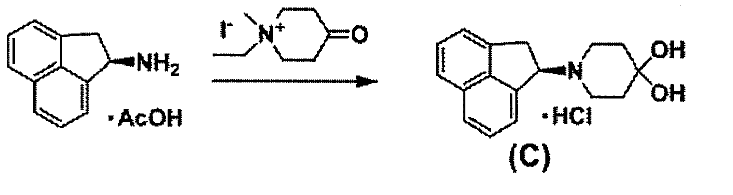

### Production of compound (C)

A suspension of (R)-(acenaphthen-1-yl)amine acetate (50 g, 218 mmol) derived from compound (G) produced in Example 2 in acetonitrile (350 g) was controlled to 70°C, and 30% aqueous sodium hydroxide solution (28.5 g, 43 mmol) was added. After stirring at the same temperature for 30 min, a solution (148 mL) of N-ethyl-N-methyl-4-oxopiperidinium iodide (16.43 g, 214 mmol) in water was added dropwise. After stirring at the same temperature for 2 hr, the reaction mixture was cooled to 30°C and partitioned. Sodium chloride (20 g) was added to the aqueous layer, and the mixture was partitioned. The organic layers were combined and the mixture was distilled away to about 150 g under reduced pressure. 35% Aqueous hydrochloric acid solution (22.7 g, 217 mmol) was added at 15°C, and the precipitated crystals were collected by filtration, washed with acetonitrile (100 g) and dried at 45°C under reduced pressure to give 1-((R)-acenaphthen-1-yl)-4,4-dihydroxypiperidine hydrochloride [compound (C)] (53.09 g, yield 79.3%).
¹H NMR (CDCl₃); 2.44 (2H, m), 2.78 (1H, m), 3.41 (1H, m), 3.65 (4H, m), 3.93 (2H, m), 5.55 (1H, m), 7.41 (1H, m), 7.58 (2H, m), 7.74 (1H, m), 7.89 (1H, m), 8.04 (1H, m)

### Example 4

In the same manner as in Example 2 and using acenaphthenone (about 500 mg) and the corresponding amine, the following compounds F1 - 6 and F (reference) were produced.

**Table 1**

| 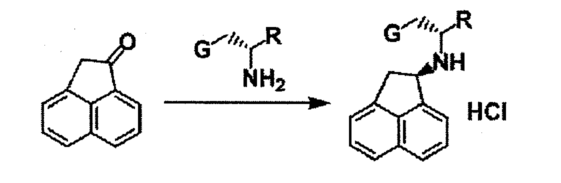 | | | | | | |
|---|---|---|---|---|---|---|
| | | | diastereomer ratio | | | |
| compound | G | R | [reaction solution] | [isolation (HCl salt)] | isolation yield (%) | LS-MS (M+1) |
| F1 | OH | phenyl | 76/24 | 90/10 | 54^{*)} | 290 |
| F2 | OH | benzyl | 98.3/1.7 | 98.3/1.7 | 44^{*)} | 3.4 |
| F3 | OH | butyl | 97.5/2.5 | 97.5/2.5 | 42^{*)} | 270 |
| F4 | OH | isopropyl | 96.3/3.7 | 96.3/3.7 | 55^{*)} | 256 |
| F5 | OH | tert-butyl | 99.3/0.7 | 99.3/0.7 | 44^{*)} | 270 |
| F6 | OH | isobutyl | 99.7/0.3 | 99.7/0.3 | 80 | - |
| F | H | phenyl | 84.16 | 84/16 | 82 | 274 |

| | | | | | | |
|---|---|---|---|---|---|---|
| ^{*)} recrystallization from 2-propanol/water | | | | | | |

The above-mentioned diastereomer ratio was analyzed using HPLC (column: Inertsil ODS-3V 5 µm 4.6x250 mm/elution solvent: gradient of 0.2% aqueous trifluoroacetic acid solution and 0.2% trifluoroacetic acid-acetonitrile solution).
Compound (F) was subjected to a hydrogenation reaction to convert to compound (G). However, many byproducts were produced, and compound (G) could not be obtained.

### Example 5

In the same manner as in Example 2 and using acenaphthenone (about 500 mg) and the corresponding amine, compound F7 having an opposite steric structure was produced.

**Table 2**

| 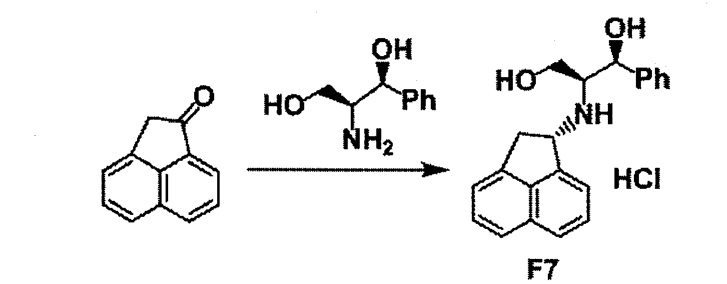 | | | |
|---|---|---|---|
| diastereomer ratio | | | |
| [reaction solution] | [isolation (HCl salt)] | isolation yield (%) | LS-MS (M+1) |
| 89/11 | 99.6/0.4 | 53^{*)} | 320 |

| | | | |
|---|---|---|---|
| ^{*)} recrystallization from 2-propanol/water | | | |

### Industrial Applicability

According to the production method of the present invention, a 1-(4-piperidinyl)benzimidazolone derivative or a salt thereof can be industrially produced in a high yield at a low cost.

## Claims

1. A method of producing a 1-(4-piperidinyl)benzimidazolone derivative of formula 1 or a salt thereof, comprising the following steps:
[step 1] a step of subjecting a piperidone compound of formula 2 and an aniline compound of formula 3 to a reductive amination reaction to give a compound of formula 4 [step 2] a step of reacting the compound of formula 4 with dit-alkyl dicarbonate or N,N'-disuccinimidyl carbonate to give the 1-(4-piperidinyl)benzimidazolone derivative of formula 1 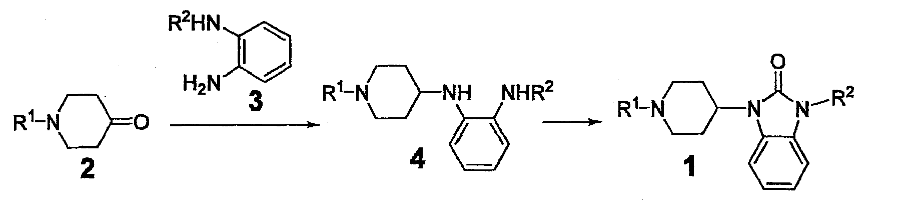
wherein R¹ is optionally substituted alkyl or an optionally substituted cyclic group, and
R² is optionally substituted alkyl, optionally substituted alkenyl or optionally substituted aryl.

2. The production method according to claim 1, wherein R¹ is any group of the following formulas (a) - (c): 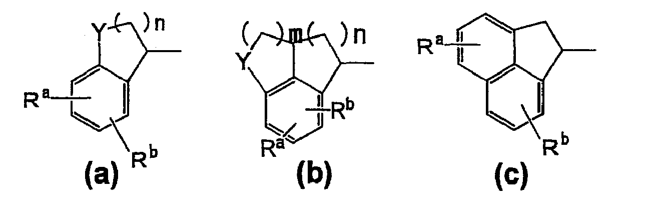
wherein m and n are the same or different and each is an integer of 1 - 3,
R^{a} and R^{b} are the same or different and each is hydrogen, alkyl, halogen, alkoxy, trifluoromethyl, trifluoromethoxy, hydroxyl, nitro, amino, alkanoylamino or cyano, and
Y is CH₂, C(CH₃)₂, O, S, SO or SO₂ and
R² is alkyl, alkenyl, alkyl-carboxyl, alkyl-C(O)O-alkyl, alkenyl-C(O)O-alkyl, alkyl-O-alkyl, alkyl-C(O)-NR³R⁴, alkyl-S-alkyl, alkyl-S(O)-alkyl, alkyl-S(O)₂-alkyl, alkyl-S(O)-NR³R⁴, alkyl-NR³R⁴, alkyl-NR⁵-C(O)-alkyl, phenyl (which is optionally substituted by alkyl, halogen, alkoxy, phenoxy or benzyloxy), or benzyl (benzene ring in benzyl is optionally substituted by alkyl, halogen, alkoxy, phenoxy or benzyloxy) wherein R³ and R⁴ are the same or different and each is hydrogen, alkyl (which is optionally substituted by C₃-C₇ cycloalkyl), cycloalkyl or alkenyl, or R³ and R⁴ may be bonded to form, together with the adjacent nitrogen atom, a saturated nitrogen-containing heterocycle (which is optionally substituted by alkyl, halogen, alkoxy, phenoxy or benzyloxy), and
R⁵ is hydrogen, alkyl or alkenyl.

3. The production method according to claim 1, wherein R¹ is acenaphthen-1-yl and R² is N-methylcarbamoylmethyl.

4. The production method according to any of claims 1 to 3, wherein a reducing agent selected from alkali borohydride, alkali triaxyborohydride and borane-amine complex, and an acid selected from formic acid, alkanoic acid, halogenated alkanoic acid, arylalkanoic acid, optionally substituted arylcarboxylic acid, optionally substituted alkylsulfonic acid and optionally substituted arylsulfonic acid are used in the reductive amination reaction of step 1.

5. The production method according to any of claims 1 to 4, wherein, in step 2, the compound of formula 4 is reacted with di-t-butyl dicarbonate to give a 1-(4-piperidinyl)benzimidazolone derivative of formula 1.

6. The production method according to any of claims 1 to 5, wherein, in step 2, a base selected from pyridine, dimethylaminopyridine and N-methylimidazole is used.

7. The production method according to any of claims 1 to 6, wherein the reaction of step 2 is performed in a carbon dioxide atmosphere.

8. A compound represented by the formula: 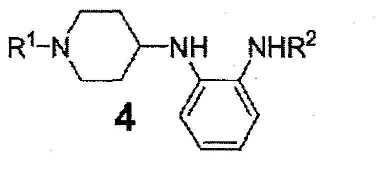 wherein R¹ and R² are as defined in claim 2,
or a salt thereof.

9. The compound according to claim 8, wherein R¹ is acenaphthen-1-yl and R² is N-methylcarbamoylmethyl, or a salt thereof.

10. A method of producing a 1-(4-piperidinyl)benzimidazolone derivative of formula 1a or a salt thereof, comprising the following steps:
[step 1] a step of reacting a ketone of formula 6 with an optically active aminoalcohol compound of formula 7 to form imine in the reaction system, and reducing the imine to give a compound of formula 8
[step 2] a step of reacting the compound of formula 8 with an oxidant to give an optically active amine of formula 5 [step 3] a step of treating the optically active amine of formula 5 with N-ethyl-N-methyl-4-oxopiperidinium iodide to give a piperidone compound of formula 2a
[step 4] a step of subjecting the piperidone compound of formula 2a and an aniline compound of formula 3 to a reductive amination reaction to give a compound of formula 4a [step 5] a step of reacting the compound of formula 4a with dialkyl dicarbonate or N,N'-disuccinimidyl carbonate to give the 1-(4-piperidinyl)benzimidazolone derivative of formula 1a 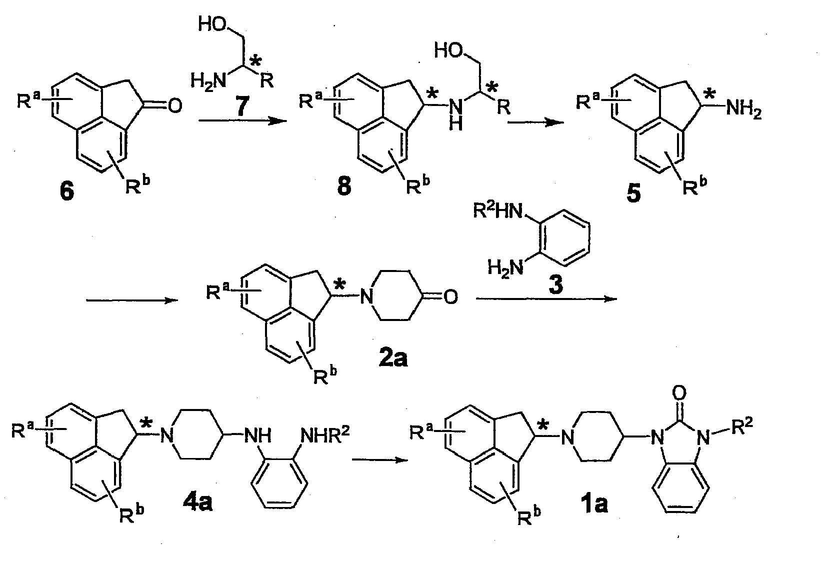
wherein R is optionally substituted C₃-C₁₀ alkyl, optionally substituted phenyl or optionally substituted benzyl,
* shows an asymmetric carbon, and
R², R^{a} and R^{b} are as defined in claim 2.
